(19) Europäisches Patentamt
European Patent Office
Office européen des brevets

(11) **EP 2 150 292 B1**

(12) **EUROPÄISCHE PATENTSCHRIFT**

(45) Veröffentlichungstag und Bekanntmachung des Hinweises auf die Patenterteilung:
**10.10.2018 Patentblatt 2018/41**

(21) Anmeldenummer: **08749204.7**

(22) Anmeldetag: **29.04.2008**

(51) Int Cl.:
*A61M 1/34* (2006.01)     *A61M 1/36* (2006.01)

(86) Internationale Anmeldenummer:
**PCT/EP2008/003439**

(87) Internationale Veröffentlichungsnummer:
**WO 2008/135193 (13.11.2008 Gazette 2008/46)**

(54) **VORRICHTUNG ZUR ÜBERWACHUNG EINER BLUTBEHANDELUNGSEINHEIT EINER EXTRAKORPORALEN BLUTBEHANDLUNGSVORRICHTUNG**

DEVICE FOR MONITORING A BLOOD TREATMENT UNIT OF AN EXTRA-CORPOREAL BLOOD TREATMENT DEVICE

DISPOSITIF DE CONTRÔLE D'UNE UNITÉ DE TRAITEMENT DU SANG D'UN DISPOSITIF DE TRAITEMENT DU SANG EXTRACORPOREL

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **04.05.2007 DE 102007020934**

(43) Veröffentlichungstag der Anmeldung:
**10.02.2010 Patentblatt 2010/06**

(73) Patentinhaber: **Fresenius Medical Care Deutschland GmbH**
**61352 Bad Homburg v.d.H. (DE)**

(72) Erfinder: **KOPPERSCHMIDT, Pascal**
**97456 Dittelbrunn (DE)**

(74) Vertreter: **Oppermann, Frank et al**
**OANDO Oppermann & Oppermann LLP**
**Washingtonstrasse 75**
**65189 Wiesbaden (DE)**

(56) Entgegenhaltungen:
**EP-A- 0 330 761      EP-A- 1 595 560**
**WO-A-03/047656      WO-A-2004/073772**
**DE-C1- 10 115 991    DE-C1- 19 734 002**
**DE-C1- 19 901 078**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft eine Vorrichtung zur Überwachung der Zunahme des Strömungswiderstandes einer durch eine semipermeable Membran in eine Blutkammer und eine Dialysierflüssigkeitskammer unterteilten Blutbehandlungseinheit für eine extrakorporale Blutbehandlungsvorrichtung sowie eine extrakorporale Blutbehandlungsvorrichtung mit einer Vorrichtung zur Überwachung der Blutbehandlungseinheit.

Zur Entfernung von harnpflichtigen Substanzen und zum Flüssigkeitsentzug werden beim akuten und chronischen Nierenversagen verschiedene Verfahren zur apparativen Blutbehandlung eingesetzt. Bei der Hämodialyse (HD) wird das Blut eines Patienten in einem extrakorporalen Blutkreislauf durch eine Kammer eines von einer semipermeablen Membran in zwei Kammern unterteilten Dialysators geleitet, während die andere Kammer von einer Dialysierflüssigkeit durchströmt wird. Über die Membran des Dialysators findet im Wesentlichen ein diffusiver Stoffaustausch statt. Bei der Hämofiltration (HF) liegt nur ein konvektiver Stoffaustausch vor. Eine Kombination aus beiden Verfahren ist die Hämodiafiltration (HDF).

Die über die Membran des Dialysators dem im extrakorporalen Blutkreislauf strömenden Blut entzogene Menge an Flüssigkeit wird als Ultrafiltrat bezeichnet. Bei der Hämodiafiltration wird ein Teil des durch die Membran des Dialysators entzogenen Ultrafiltrats durch eine sterile Substitutionsflüssigkeit ersetzt, die entweder stromauf des Dialysators (Prädilution) oder stromab des Dialysators (Postdilution) dem extrakorporalen Blutkreislauf wieder zugeführt wird. Prä- und Postdilution können auch gleichzeitig erfolgen. Das sterile Substituat, das dem Blutkreislauf zugeführt wird, kann online aus der Dialysierflüssigkeit hergestellt werden. Als Substituatrate wird diejenige Menge an Substituat bezeichnet, die in einem bestimmten Zeitraum dem im extrakorporalen Blutkreislauf strömenden Blut zugeführt wird. Als Nettoentzugsrate, im allgemeinen Sprachgebrauch auch als Ultrafiltrationsrate wird die Rate bezeichnet, mit der dem Patienten Flüssigkeit entzogen wird. Diese ergibt sich als Differenz der Substitutionsrate und der Rate der Flüssigkeitsverschiebung über die Membran.

Es hat sich gezeigt, dass eine HDF-Blutbehandlung, bei der eine Postdilution erfolgt, bei gleicher Substituatrate gegenüber einer Behandlung, bei der eine Prädilution erfolgt, eine höhere Effizienz hat. Die höhere Reinigungsleistung bei postdilutiver Zugabe von Substitutionsflüssigkeit gegenüber prädilutiver Substitution ist darauf zurückzuführen, dass bei Postdilution das Filtrat in vollem Umfang aus dem zu reinigenden Blut gewonnen wird, während bei Prädilution das mit Substituat verdünnte Blut in den Dialysator strömt (DE 103 55 042 B3).

Für eine extrakorporale Blutbehandlung ist der Strömungswiderstand der Membran des Dialysators von Bedeutung. Bei einem zu hohen Strömungswiderstand kann das zu reinigende Blut im extrakorporalen Blutkreislauf ggf. nicht mit der erforderlichen Förderrate gefördert werden, wodurch die Effektivität der Blutbehandlung verringert wird. Ein stark erhöhter Strömungswiderstand des Dialysators kann sogar einen vollständigen Verschluss der Membran zur Folge haben. Dann ist die Blutbehandlung unterbrochen, wobei gegebenenfalls das gesamte Blutschlauchsystem zu ersetzen ist (DE 103 55 042 B3). Durch die Beeinflussung der Austauschoberflächen der Membran, insbesondere auch der Poren der Membran selbst, wird die Effektivität der Blutbehandlung selbst bei unveränderter Förderrate verringert.

Die DE 103 55 042 B3 beschreibt ein Verfahren zum Erkennen von Störungen des Blutflusses in einem extrakorporalen Blutkreislauf während einer extrakorporalen Blutbehandlung mit einer extrakorporalen Blutbehandlungsvorrichtung. Das bekannte Verfahren beruht auf der Analyse eines sich im extrakorporalen Blutkreislauf fortpflanzenden oszillierenden Drucksignals, das gemessen und analysiert wird, wobei der Phasenwinkel mindestens einer Oberschwingung des Drucksignals ermittelt wird. Eine Störung des Blutflusses im extrakorporalen Blutkreislauf wird auf der Grundlage der Veränderung des Phasenwinkels der mindestens einen Oberschwingung detektiert.

Aus der WO 2004/073772 A1 ist ein Verfahren bekannt, mit dem das Zusetzen der Membran eines Dialysators detektiert werden kann. Das bekannte Verfahren beruht auf einer Analyse des Frequenzspektrums eines über den Dialysator übertragenen Drucksignals. Während der Blutbehandlung werden die Druckverhältnisse im extrakorporalen Blutkreislauf und/oder im Dialysierflüssigkeitssystem kontinuierlich überwacht. Während der Druck im extrakorporalen Kreislauf und/oder Dialysierflüssigkeitssystem zur Bestimmung des Strömungswiderstandes des Dialysators gemessen wird, bleibt die Substituatrate und die Ultrafiltrationsrate unverändert.

Die US 2002/0174721 A1 und die US 6,623,443 B1 beschreiben Verfahren zur Erkennung von Stenosen in einem Schlauchleitungssystem eines extrakorporalen Blutkreislaufs. Die beiden Verfahren beruhen auf einer Analyse von Druckpulsen, die im extrakorporalen Blutkreislauf detektiert werden. Das aus der US 2002/0174721 A1 bekannte Verfahren sieht vor, das Frequenzspektrum der Druckpulse zu analysieren und die Dämpfung mindestens einer Oberschwingung des Drucksignals zu ermitteln, wobei auf eine Stenose bei einer Änderung der Dämpfung geschlossen wird. Eine Änderung der Substituat- oder Ultrafiltrationsrate findet bei der Analyse der Druckpulse keine Berücksichtigung.

Die WO 03/047656 A1 offenbart die Präambel des Anspruchs 1 und beschreibt ein Verfahren zur Überwachung der Zufuhr von Substitutionsflüssigkeit während einer extrakorporalen Blutbehandlung. Die Überwachung der Zufuhr von Substitutionsflüssigkeit beruht darauf, dass von der Substituatpumpe erzeugte Druckwellen im extrakorporalen Blutkreislauf gemessen werden. Eine Auswerteinheit stellt fest, ob eine Störung der Substitutionsflüssigkeitszufuhr vorliegt.

Aus der EP 1 595 560 A1 ist ebenfalls ein Verfahren zur Überwachung der Zufuhr von Substitutionsflüssigkeit bekannt. Bei dem bekannten Verfahren soll zwischen einer Prä- und Postdilution unterschieden werden. Die Erkennung der Prä- oder Postdilution erfolgt auf der Grundlage der Veränderung des Drucks nach dem Abschalten und/oder Einschalten der für die Förderung der Substitutionsflüssigkeit vorgegebenen Substituatpumpe.

Aus der EP 0 330 761 A1 ist ein Verfahren zur Überwachung von Flüssigkeitssystemen medizinischer Geräte bekannt, bei dem nicht nur der statische Druck, sondern auch Druckschwankungen überwacht werden. Die DE 197 34 002 C1 beschreibt ein Verfahren zur Überwachung eines Gefäßzugangs während einer Dialysebehandlung, bei dem im extrakorporalen Blutkreislauf Druckpulse überwacht werden, um auf das Herausrutschen der Nadel zu schließen.

**Der** Erfindung **liegt** die Aufgabe zu Grunde, eine Vorrichtung zur Überwachung einer Blutbehandlungseinheit einer extrakorporalen Blutbehandlungsvorrichtung zu schaffen, die eine Bestimmung einer für die Aufrechterhaltung des Blutflusses im extrakorporalen Kreislauf oder die Reinigungsleistung der Blutbehandlungseinheit aussagekräftigen Größe ermöglicht. Eine weitere Aufgabe der Erfindung ist, eine Blutbehandlungsvorrichtung mit einer derartigen Vorrichtung zur Überwachung der Blutbehandlungseinheit bereitzustellen.

Die Lösung dieser Aufgaben erfolgt erfindungsgemäß mit den Merkmalen der Patentansprüche **1 bzw. 8.** Vorteilhafte Ausführungsformen der Erfindung sind Gegenstand der Unteransprüche.

Die erfindungsgemäße Vorrichtung setzt voraus, dass dem Blut im extrakorporalen Blutkreislauf mit einer vorgegebenen Substituatrate, die größer oder gleich Null sein kann, Substituat stromauf oder stromab der Blutbehandlungseinheit zugeführt werden kann bzw. kein Substituat zugeführt wird, und/oder über die semipermeable Membran der Blutbehandlungseinheit mit einer vorgegebenen Ultrafiltrationsrate, die wiederum größer oder gleich Null sein kann, Ultrafiltrat entzogen werden kann bzw. kein Ultrafiltrat entzogen wird.

Die erfindungsgemäße Vorrichtung beruht im Wesentlichen darauf, dass im Dialysator oder Filter unterschiedliche Bedingungen geschaffen werden, bei denen jeweils eine Messung erfolgt. Dies kann insbesondere durch eine Veränderung der Viskosität des Bluts stromauf des Dialysators oder Filters (Prädilution) und/oder eine Veränderung der Viskosität des Bluts im Dialysator oder Filter erfolgen. Dabei kann die Veränderung der Viskosität des Bluts dadurch herbeigeführt werden, dass dem Blut im extrakorporalen Kreislauf Substituat zugeführt wird und/oder über die semipermeable Membran des Dialysators oder Filters Ultrafiltrat entzogen wird. Eine Veränderung der Substituat- und/oder Ultrafiltrationsrate führen also zu einer Veränderung der Viskosität des Bluts.

Die Überwachung der Blutbehandlungseinheit der extrakorporalen Blutbehandlungsvorrichtung beruht auf zwei Messungen zu unterschiedlichen Zeitpunkten.

Die erste Messung erfolgt zu einem Zeitpunkt, wenn dem extrakorporalen Blutkreislauf Substituat mit einer vorgegebenen ersten Substituatrate bzw. kein Substituat stromauf oder stromab der Blutbehandlungseinheit zugeführt wird, und/oder über die semipermeable Membran der Blutbehandlungseinheit Ultrafiltrat mit einer vorgegebenen ersten Ultrafiltrationsrate bzw. kein Ultrafiltrat entzogen wird, und die zweite Messung erfolgt, wenn dem extrakorporalen Blutkreislauf Substituat mit einer vorgegebenen zweiten Substituatrate bzw. kein Substituat zugeführt wird, wobei sich die zweite Substituatrate von der ersten Substituatrate unterscheidet, und/oder über die Membran der Blutbehandlungseinheit Ultrafiltrat mit einer vorgegebenen zweiten Ultrafiltrationsrate bzw. kein Ultrafiltrat entzogen wird, wobei sich die zweite Ultrafiltrationsrate von der ersten Ultrafiltrationsrate unterscheidet. Dabei kann die Substituatrate und Ultrafiltrationsrate größer oder gleich Null sein.

Es ist unerheblich, ob zuerst die erste oder die zweite Messung erfolgt. Allein entscheidend ist, dass bei beiden Messungen unterschiedliche Substituatraten und/oder Ultrafiltrationsraten eingestellt sind. Beispielsweise kann die Substituatrate um einen vorgegebenen Wert erhöht oder verringert werden. Der einfachste Fall ist, dass bei der ersten Messung die Blutbehandlungsvorrichtung mit einer vorgegebenen Substituatrate und/oder Ultrafiltrationsrate betrieben wird, die größer als Null ist, wobei für die zweite Messung die Substitution von Substituat bzw. der Entzug von Ultrafiltrat unterbrochen wird. Alternativ ist auch möglich, bei der ersten Messung die Substitution und/oder Ultrafiltration zu unterbrechen und bei der zweiten Messung die Blutbehandlungsvorrichtung mit einer vorgegebenen Substituatrate und/oder Ultrafiltrationsrate zu betreiben.

Auf der Grundlage der gemessenen oszillierenden Drucksignale vor und nach der Änderung der Substituatrate und/oder Ultrafiltrationsrate wird eine mit der Änderung des Strömungswiderstandes der Blutbehandlungseinheit korrelierende Größe berechnet.

Bei einer bevorzugten Ausführungsform wird mit einer Änderung der Substituatrate für die erste und zweite Messung auch eine Änderung der Ultrafiltrationsrate vorgenommen, mit der dem extrakorporalen Blutkreislauf eine vorgegebene Menge an Ultrafiltrat entzogen wird. In diesem Zusammenhang wird nachfolgend unter Ultrafiltrationsrate nicht die "Nettoentzugsrate", sondern die Rate verstanden, mit der Flüssigkeit über die Membran des Dialysators oder Filters verschoben wird.

Zu dem Zeitpunkt, zu dem Substituat mit der vorgegebenen ersten Substituatrate zugeführt wird, wird eine erste Ultrafiltrationsrate eingestellt, während zu dem Zeitpunkt, zu dem Substituat mit der zweiten Substituatrate zugeführt wird, eine zweite Ultrafiltrationsrate eingestellt wird. Die Ultrafiltrationsrate wird vorzugsweise um den gleichen Betrag erhöht bzw. verringert, wie die Substituatrate erhöht bzw. verringert wird. Vorzugsweise sollte die Erhöhung bzw. Verringerung von Substituat- und Ultrafiltrationsrate gleichzeitig erfolgen. Dies ist aber nicht zwingend erforderlich. So kann zwischen der Veränderung der Substituat- und Ultrafiltrationsrate ein gewisser Zeitraum liegen.

Die mit der Änderung des Strömungswiderstandes korellierende Größe kann mit einem vorgegebenen Grenzwert verglichen werden, wobei auf einen kritischen Zustand geschlossen wird, wenn die mit der Änderung des Strömungswiderstandes korellierende Größe den vorgegebenen Grenzwert überschreitet.

Für den Fall, dass auf einen kritischen Zustand geschlossen wird, kann ein Eingriff in die Blutbehandlung vorgenommen werden, um dem kritischen Zustand entgegenzuwirken. Beispielsweise kann die Substituatrate oder Ultrafiltrationsrate verändert werden. Auf jeden Fall ist zu vermeiden, dass sich die Membran der Blutbehandlungseinheit zusetzt.

Es ist auch möglich, die mit der Änderung des Strömungswiderstandes korrelierende Größe dahingehend zu überwachen, dass sie mit einem vorgegebenen Grenzwert verglichen wird, nach dessen Überschreitung ein akustischer und/oder optischer Alarm gegeben wird.

Eine bevorzugte Ausführungsform sieht vor, dass die Berechnung der mit der Änderung des Strömungswiderstandes korellierenden Größe auf einer Analyse des Frequenzspektrums des vor der Änderung der Substituatrate gemessenen oszillierenden Drucksignals und nach der Änderung der Substituatrate gemessenen oszillierenden Drucksignals erfolgt, wobei die Änderung der Amplitude der Grundschwingung und/oder die Änderung der Amplitude mindestens einer Oberschwingung des vor und nach der Änderung der Substituatrate gemessenen oszillierenden Drucksignals bestimmt wird. Die Änderung des Strömungswiderstandes kann dann auf der Grundlage der Änderung der Amplitude der Grundschwingung und/oder der mindestens einen Oberschwingung berechnet werden. In der Praxis kann es ausreichend sein, dass nur die Amplitudenänderung der Grundschwingung ausgewertet wird.

Die Analyse der gemessenen Drucksignale erfolgt vorzugsweise mit einer Fourier-Transformation. Es sind aber auch andere Verfahren möglich, die dem Fachmann bekannt sind, beispielsweise das Least-Square-Verfahren, mit dem versucht wird, die Messwerte durch eine angepasste Linearkombination von Basisfunktionen wiederzugeben.

Für die erfindungsgemäße Vorrichtung ist grundsätzlich nicht relevant, wie die oszillierenden Drucksignale im extrakorporalen Blutkreislauf erzeugt werden. Von Vorteil ist, wenn die oszillierenden Druckpulse ausgewertet werden, die von der im extrakorporalen Blutkreislauf angeordneten Blutpumpe, insbesondere einer okkludierenden Blutpumpe, erzeugt werden.

Darüber hinaus ist es für die Bestimmung der mit der Änderung des Strömungswiderstandes korellierenden Größe grundsätzlich möglich, die oszillierenden Drucksignale im extrakorporalen Blutkreislauf oder im Dialysierflüssigkeitssystem zu messen, da eine Änderung des Strömungswiderstandes im Allgemeinen sowohl mit einer longitudinalen als auch lateralen Komponente einhergeht.

Bei den bekannten Blutbehandlungsvorrichtungen ist die ein oszillierendes Drucksignal erzeugende okkludierende Blutpumpe, insbesondere Rollenpumpe, im Allgemeinen im arteriellen Zweig des extrakorporalen Blutkreislaufs angeordnet. Die oszillierenden Druckpulse der Blutpumpe, die im extrakorporalen Blutkreislauf die Blutkammer der Blutbehandlungseinheit durchlaufen, können als oszillierendes Drucksignal im venösen Zweig des extrakorporalen Kreislaufs gemessen werden. Dieses Drucksignal ist für die Änderung des Strömungswiderstandes längs der Fasern des Dialysators repräsentativ. Die über die Membran der Blutbehandlungseinheit übertragenen Druckpulse, die für den Strömungswiderstand quer zu den Fasern des Dialysators charakteristisch sind, können als oszillierende Drucksignale im Dialysierflüssigkeitssystem gemessen werden. Vorzugsweise werden die Druckpulse stromab der Blutbehandlungseinheit in der Dialysierflüssigkeitsabführleitung nachgewiesen. Die Druckpulse können aber grundsätzlich auch in der Dialysierflüssigkeitszuführleitung gemessen werden.

Die erfindungsgemäße Vorrichtung zur Überwachung der Blutbehandlungseinheit kann eine separate Einrichtung oder Bestandteil der extrakorporalen Blutbehandlungsvorrichtung sein. Da einzelne Komponenten der erfindungsgemäßen Überwachungsvorrichtung bereits in den bekannten Blutbehandlungsvorrichtungen enthalten sind, bietet sich eine Integration in die Blutbehandlungsvorrichtung an. Beispielsweise verfügen die bekannten Dialysevorrichtungen im Allgemeinen über Drucksensoren im extrakorporalen Blutkreislauf und im Dialysierflüssigkeitssystem. Insofern kann die erfindungsgemäße Überwachungsvorrichtung ohne größeren Aufwand an Hardware in die bekannten Dialysevorrichtungen implementiert werden.

Im Folgenden wird ein Ausführungsbeispiel der Erfindung unter Bezugnahme auf die Zeichnungen näher erläutert.

Es zeigen:

Fig. 1  ein vereinfachtes elektrisches Ersatzschaltbild zur Darstellung der Strömungsverhältnisse an einer Blutbehandlungseinheit einer extrakorporalen Blutbehandlungsvorrichtung,

Fig. 2  die wesentlichen Komponenten einer erfindungsgemäßen extrakorporalen Blutbehandlungsvorrichtung zusammen mit einer erfindungsgemäßen Vorrichtung zur Überwachung der Blutbehandlungseinheit der Blutbehandlungsvorrichtung in vereinfachter schematischer Darstellung,

Fig. 3  das Quadrat der Amplitude der Grundschwingung sowie der ersten und zweiten Oberschwingung des gemes-

Fig. 4 senen oszillierenden Drucksignals als Funktion der Behandlungszeit bei einer Hämodiafiltration mit Postdilution, das Quadrat der Amplitude der Grundschwingung sowie der ersten und zweiten Oberschwingung des oszillierenden Drucksignals als Funktion der Behandlungszeit bei einer Hämodiafiltration mit Prädilution,

Fig. 5 eine Tabelle, aus der die Änderung des Strömungswiderstandes der Blutbehandlungseinheit bei einer Hämodiafiltration (HDF) mit Postdilution während der Blutbehandlung ersichtlich ist und

Fig. 6 eine Tabelle, aus der eine Strömungswiderstandsänderung bei einer Hämodiafilatration (HDF) mit Prädilution während der Blutbehandlung ersichtlich ist.

Nachfolgend werden die theoretischen Grundlagen eines Verfahrens unter Bezugnahme auf einen durch eine semipermeable Membran in eine Dialysierflüssigkeitskammer und eine Blutkammer unterteilten Dialysators beschrieben, wobei die Blutkammer des Dialysators in einem extrakorporalen Blutkreislauf und die Dialysierflüssigkeitskammer in einem Dialysierflüssigkeitssystem angeordnet sind.

Der longitudinale Strömungswiderstand im Dialysator, d.h. der Strömungswiderstand längs der Fasern der Membran des Dialysators auf der Blutseite, hängt primär von der Fließgeschwindigkeit des Blutes durch die Fasern des Dialysators, der Viskosität des durch die Blutkammer des Dialysators strömenden Bluts, die dem lokalen Hämatokrit im Dialysator äquivalent ist, sowie dem Querschnitt und der Länge der Fasern ab.

Bei der Hämodiafiltrationsbehandlung (HDF) wird durch einen erhöhten konvektiven Transport Blutwasser über die Dialysatormembran auf die Dialysierflüssigkeitsseite überführt, während zur Wahrung der Volumenbilanz prädilutiv, d.h. stromaufwärts des Dialysators oder postdilutiv, stromabwärts des Dialysators, Substituat substituiert wird. Der Strömungswiderstand des zu dialysierenden Bluts längs der Dialysatorfasern wird durch den konvektiven Entzug stark beeinflusst. Ein hoher Strömungswiderstand im Dialysator kann zu einem dem Okklusionsdruck der Blutpumpe überschreitenden Filtereingangsdruck führen, so dass die Gefahr der mechanischen Hämolyse besteht, oder zu einem vollständigen Verschluss der Fasern im Dialysator, was auch als Klotten des Dialysators bezeichnet wird. Die Eindickung des zu dialysierenden Bluts durch einen hohen konvektiven Wasserentzug führt auch lateral zu den Dialysatorfasern zu einem erhöhten Strömungswiderstand. Die konvektive Entzugsmenge, die der Substituatrate entspricht, sollte idealerweise so gewählt werden, dass ein möglichst großer konvektiver Transport bei noch stabilem, nicht divergentem Strömungswiderstand im Dialysator möglich ist.

Die Erfindung schlägt eine Messgröße vor, die der Änderung des dynamischen longitudinalen bzw. lateralen Strömungswiderstandes des Dialysators einer Hämodiafiltrationsbehandlung im Vergleich zu einer Hämodialysebehandlung entspricht.

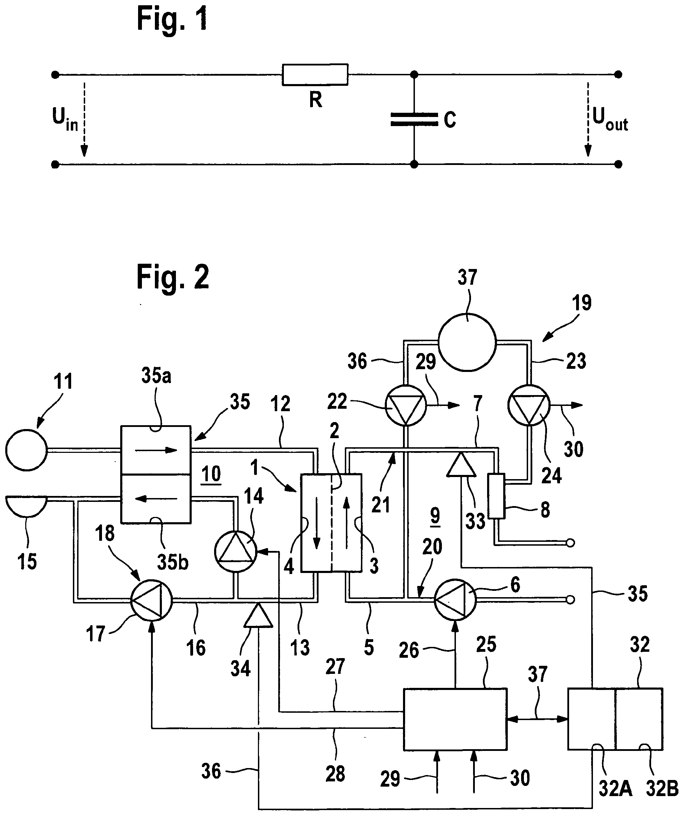

Die Strömungsverhältnisse am Dialysator können mit dem vereinfachten elektrischen Schaltbild von Fig. 1 beschrieben werden. Gemäß der vereinfachten Darstellung der Strömungsverhältnisse am Dialysator mittels der elektrischen Analogie fungiert der Dialysator als Tiefpass für die von der Blutpumpe generierten Druckpulse ($U_{in}$). Dieser Tiefpass ist durch das Produkt RC definiert. Der in der Analogie als R bezeichnete Widerstand ist dem longitudinalen Strömungswiderstand des Dialysators äquivalent.

Ein oszillierendes Eingangssignal $U_{in}$ führt zu einem frequenzabhängigen gedämpften Ausgangssignal $U_{out}$. Der Zusammenhang zwischen $U_{in}$ und $U_{out}$ mit $\omega$ als Periodizität des Eingangssignals $U_{in}$ lautet:

$$U_{out}(\omega t) = \frac{U_{in}(\omega t)}{1 + i\omega RC} \ , \qquad\qquad (1)$$

wobei i die komplexe Einheit bezeichnet.

Ein mit der Frequenz $\omega$ oszillierendes Eingangssignal $U_{in}$ führt gemäß Gleichung (1) zu einer Amplitudendämpfung des Ausgangssignals $U_{out}$:

$$U_{in}(\omega t) = (A+iB) \cdot e^{i\omega t}$$

$$U^{*}_{in}(\omega t) = (A-iB) \cdot e^{-i\omega t}$$

$$U^{2}_{out} = U_{out}(\omega t) \cdot U^{*}_{out}(\omega t) = \frac{(A+iB) \cdot e^{i\omega t}}{1+i\omega RC} \cdot \frac{(A-iB) \cdot e^{-i\omega t}}{1-i\omega RC} = \frac{A^{2}+B^{2}}{1+\omega^{2}R^{2}C^{2}} \ , \quad (2)$$

wobei die komplex konjugierte Darstellung von U mit U* bezeichnet ist.

Die interessierende Größe des longitudinalen Dialysatorströmungswiderstandes bzw. der longitudinalen Dialysatorimpedanz lautet R($\omega$). Elementare Umformungen liefern den Zusammenhang:

$$R(\omega) = \frac{1}{\omega C} \sqrt{\left( \frac{U_{in}^2(\omega)}{U_{out}^2(\omega)} - 1 \right)} \quad , \tag{3}$$

wobei mit $U_{in}^2$ das Absolutquadrat der Amplitude des Eingangssignals $U_{in}$ und mit $U_{out}^2$ das Absolutquadrat der Amplitude des Ausgangssignals $U_{out}$ bezeichnet wird.

Ändert sich das Amplitudenquadrat der erhaltenen Signale $U_{out}$ um $\Delta U_{out}^2$, so ändert sich gemäß Gleichung (3) der Widerstand R um $\Delta$R.

$$\frac{R + \Delta R}{R} = \sqrt{\frac{\left( \frac{U_{in}^2(\omega)}{U_{out}^2(\omega) - \Delta U_{out}^2(\omega)} - 1 \right)}{\left( \frac{U_{in}^2(\omega)}{U_{out}^2(\omega)} - 1 \right)}} =$$

$$= \sqrt{\frac{U_{in}^2(\omega) - U_{out}^2(\omega) + \Delta U_{out}^2(\omega)}{U_{out}^2(\omega) - \Delta U_{out}^2(\omega)} \cdot \frac{U_{out}^2(\omega)}{U_{in}^2(\omega) - U_{out}^2(\omega)}} =$$

$$= \sqrt{\frac{U_{out}^2(\omega)}{U_{out}^2(\omega) - \Delta U_{out}^2(\omega)} \left( 1 + \frac{\Delta U_{out}^2(\omega)}{U_{in}^2(\omega) - U_{out}^2(\omega)} \right)} \tag{4}$$

Die Änderung des Strömungswiderstandes ($\Delta$R +R)/R hängt demnach von dem Amplitudenquadrat des Eingangssignals $U_{in}$ ab, das jedoch im Allgemeinen nicht bekannt ist. Unter der plausiblen Annahme, dass die Dämpfung des Drucksignals groß ist, gilt näherungsweise:

$$\frac{\Delta U_{out}^2(\omega)}{U_{in}^2(\omega) - U_{out}^2(\omega)} << 1 \, , \tag{5}$$

so dass gemäß Gleichung (4) folgt:

$$\frac{R + \Delta R}{R} \approx \sqrt{\frac{U_{out}^2(\omega)}{U_{out}^2(\omega) - \Delta U_{out}^2(\omega)}} \, . \tag{6}$$

Aus den ermittelten Signalintensitäten und deren Veränderung kann also auf die Änderung der relativen Strömungsimpedanz längs der Dialysatorfasern bei einer bestimmten Frequenz der Anregung, d.h. dem Vielfachen der Drehzahl der Blutpumpe, geschlossen werden. Zur Berechnung der Strömungsimpedanz lateral zu den Dialysatorfasern werden die veränderten Signalintensitäten bei Übergang von der Blutseite zur Dialysierflüssigkeitsseite ermittelt.

Falls die oben angenommene Bedingungen (Gleichung 5) nicht erfüllt ist, muss $U_{in}^2 (\omega)$ bestimmt werden. Das Amplitudenquadrat des Eingangssignals kann dadurch bestimmt werden, dass im extrakorporalen Blutkreislauf stromauf des Dialysators das oszillierende Drucksignal der Blutpumpe gemessen und der Realteil der spektralen Komponente des Drucksignals berechnet wird. Es ist alternativ aber auch möglich, den RC-Anteil der elektrischen Leistungsaufnahme der Blutpumpe zu bestimmen.

Für die Ausführung der erfindungsgemäßen Lehre nicht es grundsätzlich nicht erfoderlich, die Größe "R" zu bestimmen. Im Prinzip kann bereits die Größe "U" bzw. "U$^2$" ausgewertet werden, um die Blutbehandlungseinheit zu überwachen.

Fig. 2 zeigt die wesentlichen Komponenten der erfindungsgemäßen Blutbehandlungsvorrichtung zusammen mit der erfindungsgemäßen Überwachungsvorrichtung. Bei der Blutbehandlungsvorrichtung handelt es sich um eine Hämodiafiltrationsvorrichtung, die als Blutbehandlungseinheit einen Dialysator oder Filter 1 aufweist, der durch eine semipermeable Membran 2 in eine Blutkammer 3 und eine Dialysierflüssigkeitskammer 4 getrennt ist. Der Einlass der Blutkammer 3 ist mit einem Ende einer Blutzuführleitung 5 verbunden, in die eine Blutpumpe 6, insbesondere eine Druckpulse erzeugende Rollenpumpe, geschaltet ist, während der Auslass der Blutkammer mit einem Ende einer Blutabführleitung 7 verbunden ist, in die eine Tropfkammer 8 geschaltet ist. Blutzuführ- und -abführleitung 5, 7 bilden mit der Blutkammer 3 des Dialysators den extrakorporalen Blutkreislauf 9 der Hämodiafiltrationsvorrichtung. Bei der Blutzuführ- und -abführleitung 5, 7 handelt es sich um Schlauchleitungen eines in die Hämodiafiltrationsvorrichtung eingelegten Schlauchsets (Disposable).

Das Dialysierflüssigkeitssystem 10 der Hämodiafiltrationsvorrichtung umfasst eine Einrichtung 11 zur Bereitstellung von Dialysierflüssigkeit, die über den ersten Abschnitt einer Dialysierflüssigkeitszuführleitung 12 mit dem Einlass der ersten Kammerhälfte 35a einer Bilanziereinrichtung 35 verbunden ist. Der zweite Abschnitt der Dialysierflüssigkeitszuführleitung 12 verbindet den Auslass der ersten Bilanzierkammerhälfte 35a mit dem Einlass der Dialysierflüssigkeitskammer 4. Der Auslass der Dialysierflüssigkeitskammer 4 ist über den ersten Abschnitt einer Dialysierflüssigkeitsabführleitung 13 mit dem Einlass der zweiten Bilanzierkammerhälfte 35b verbunden. In den ersten Abschnitt der Dialysierflüssigkeitsabführleitung 13 ist eine Dialysierflüssigkeitspumpe 14 geschaltet. Der Auslass der zweiten Bilanzierkammerhälfte 35b ist über den zweiten Abschnitt der Dialysierflüssigkeitsabführleitung 13 mit einem Auslauf 15 verbunden. Stromauf der Dialysierflüssigkeitspumpe 14 zweigt von der Dialysierflüssigkeitsabführleitung 13 eine Ultrafiltratleitung 16 ab, die ebenfalls zu dem Auslauf 15 führt. In die Ultrafiltratleitung 16 ist eine Ultrafiltrationspumpe 17 geschaltet. Die Bilanziereinrichtung 35 besteht in handelsüblichen Geräten aus zwei parallelen Bilanzkammern, die anti-zyklisch betrieben werden. Aus Gründen der Vereinfachung braucht hierzu aber an dieser Stelle nicht näher eingegangen zu werden.

Während der Dialysebehandlung wird die Blutkammer 3 von dem Blut des Patienten und die Dialysierflüssigkeitskammer 4 des Dialysators von der Dialysierflüssigkeit durchströmt. Die Bilanziereinrichtung 35 stellt sicher, dass nur so viel Dialysierflüssigkeit über die Dialysierflüssigkeitszuführleitung zufließen kann, wie Dialysierflüssigkeit über die Dialysierflüssigkeitsabführleitung abfließen kann. Mit der Ultrafiltrationspumpe 17 kann dem Patienten eine vorgegebene Menge an Flüssigkeit (Ultrafiltrat) mit einer vorgegebenen Ultrafiltrationsrate entzogen werden. Die Ultrafiltrationspumpe 17 ist somit Teil einer Einrichtung zum Entziehen von Flüssigkeit aus dem im extrakorporalen Kreislauf 9 strömenden Blut durch die Membran 2 des Dialysators 1, die als Ultrafiltrationseinrichtung 18 bezeichnet wird.

Anstelle der in Fig. 2 gezeigten Anordnung sind auch andere Bilanziereinrichtungen gebräuchlich. Entscheidend ist, dass die Flüssigkeitszufuhr zum Dialysator 1 oder Blutkreislauf sowie die Flüssigkeitsabfuhr vom Dialysator kontrolliert wird.

Um dem Patienten die Flüssigkeit wieder zuzuführen, verfügt die Hämodiafiltrationsvorrichtung über eine Substitutionseinrichtung 19, mit der eine Substitutionsflüssigkeit (Substituat) dem Blut zugeführt werden kann, das durch den arteriellen Zweig 20 (Prädilution) und/oder den venösen Zweig 21 (Postdilution) des extrakorporalen Blutkreislaufs 9 strömt. Die Substitutionseinrichtung 19 weist eine Einrichtung 37 zur Bereitstellung von Substituat auf, von der eine erste Substituatleitung 36, in die eine erste Substituatpumpe 22 geschaltet ist, zu dem Abschnitt der Blutzuführleitung 5 zwischen Blutpumpe 6 und Blutkammer 3 führt. Eine zweite Substituatleitung 23, in die eine zweite Substituatpumpe 24 geschaltet ist, führt von der Einrichtung 37 zur Bereitstellung von Substituat zu der Tropfkammer 8. Wenn die Hämodiafiltrationsvorrichtung nur mit Postdilution bzw. Prädilution betrieben werden soll, kann die eine oder andere Substituatpumpe zusammen mit der jeweiligen Substituatleitung entfallen.

Darüber hinaus weist die Hämodiafiltrationsvorrichtung eine zentrale Steuer- und Recheneinheit 25 auf, die über Steuerleitungen 26 bis 30 mit der Blutpumpe 6, der Dialysierflüssigkeitspumpe 14, der Ultrafiltrationspumpe 17 sowie der ersten und zweiten Substituatpumpe 22, 24 verbunden ist.

Die erfindungsgemäße Vorrichtung zur Überwachung des Dialysators wird nachfolgend als Bestandteil der Blutbehandlungsvorrichtung beschrieben, da die Blutbehandlungsvorrichtung bereits über die erforderliche Hardware verfügt. Die erfindungsgemäße Vorrichtung kann grundsätzlich aber auch eine separate Einheit bilden.

Die Überwachungsvorrichtung verfügt über Mittel zum Messen von oszillierenden Drucksignalen und Mittel zum Analysieren der Drucksignale, die eine Rechen- und Auswerteinheit 32, die auch Bestandteil der zentralen Steuer- und Recheneinheit 25 sein kann, sowie einen stromab der Blutkammer 3 an der Blutabführleitung 7 angeordneten Drucksensor 33 und einen stromab der Dialysierflüssigkeitskammer 4 des Dialysators 1 stromauf der Dialysierflüssigkeitspumpe 14 an der Dialysierflüssigkeitsabführleitung 13 angeordneten Drucksensor 34 umfassen. Die Drucksensoren 33 und 34 sind über Datenleitungen 35, 36 mit der Rechen- und Auswerteinheit 32 verbunden, die über eine Datenleitung 37 mit der zentralen Steuer- und Recheneinheit 25 die erforderlichen Daten untereinander austauscht. Die zentrale Steuer- und Recheneinheit 25 kann einen Eingriff in die Maschinensteuerung dann vornehmen, wenn die Rechen- und Auswerteinheit 32 einen Störfall detektiert hat. Nachfolgend wird die Funktion der Überwachungsvorrichtung im Einzelnen beschrieben.

Die Rechen- und Auswerteinheit 32 verfügt über eine Fourier-Analyseeinrichtung 32A, die entweder das Ausgangssignal des im Blutkreislauf 9 angeordneten Drucksensors 33 oder des Drucksensors 34 im Dialysierflüssigkeitskreislauf 10 analysiert.

Die Blutpumpe 6 erzeugt oszillierende Druckpulse, die sich einerseits über die Blutzuführleitung 5 in Längsrichtung der Fasern der Membran 2 des Dialysators 1 und die Blutabführleitung 7 fortpflanzen und von dem Drucksensor 33 gemessen werden, und andererseits sich in lateraler Richtung zur Blutströmung im Dialysator ausbreiten und über die Dialysierflüssigkeitsabführleitung 13 fortpflanzen und von dem Drucksensor 34 gemessen werden.

Die Fourier-Analyseeinrichtung 32A zerlegt die oszillierenden Drucksignale des Drucksensors 32 oder des Drucksensors 33 durch eine Fourier-Analyse in die Grundschwingung und mehrere Oberschwingungen, beispielsweise die erste und zweite Harmonische.

Zunächst wird angenommen, dass die Hämodiafiltrationsvorrichtung mit einer Postdilution betrieben wird, wobei die Substituatpumpe 24 läuft und die Substituatpumpe 22 stillsteht. Die Steuer- und Recheneinheit 25 stellt die Förderrate der Substituatpumpe 24 derart ein, dass dem Blut im Blutkreislauf eine vorgegebene Menge an Substituat mit einer vorgegebenen Substituatrate, beispielsweise 20 1 Substituat während der gesamten Blutbehandlung zugeführt wird. Die Ultrafiltratpumpe 17 betreibt die Steuer- und Recheneinheit 25 mit einer Förderrate, dass sich eine Ultrafiltrationsrate einstellt, die der Höhe der Substituatrate, beispielsweise 16 1 pro Behandlung entspricht, d.h. die Menge an Ultrafiltrat, das mit der Pumpe 17 dem Dialysierflüssigkeitssystem entzogen wird, wird durch die gleiche Menge an Substituat ausgeglichen, die mit der Pumpe 24 dem Blutkreislauf zugeführt wird. Dabei wird insgesamt während der Behandlung dem Patienten beispielsweise 41 Flüssigkeit entzogen.

Mit dem venösen Drucksensor 33 werden nun die Druckpulse in der Blutrückführleitung 7 gemessen und das venöse Drucksignal mit der Fourier-Analyseeinrichtung 32A der Rechen- und Auswerteinheit 32 in die Grundschwingung sowie die erste und zweite Oberschwingung zerlegt. Die Rechen- und Auswerteinheit 32 berechnet die Amplituden der Grundschwingung sowie der ersten und zweiten Harmonischen und berechnet aus der Amplitude der Grundschwingung sowie der ersten und zweiten Harmonischen jeweils das Amplitudenquadrat ($U^2_{out}(\omega)$).

Daraufhin wird kurzzeitig die Substituatpumpe 24 gestoppt, so dass dem Blutkreislauf kein Substituat zugeführt wird. Es ist aber auch möglich, die Fördermenge der Substituatpumpe entweder kurzzeitig zu erhöhen oder zu verringern. Während die Substituatpumpe 24 angehalten ist oder die Fördermenge der Substituatpumpe erhöht oder verringert ist, wird die Ultrafiltrationspumpe 17 derart betrieben, dass sich die Ultrafiltrationsrate um den gleichen Betrag erhöht oder verringert, wie sich die Substituatrate erhöht bzw. verringert hat. Die Auswert- und Recheneinheit 32 zerlegt das Drucksignal des Drucksensors 33 bei stehender Substituatpumpe 24 wieder in die Grundschwingung und die erste und zweite Oberschwingung.

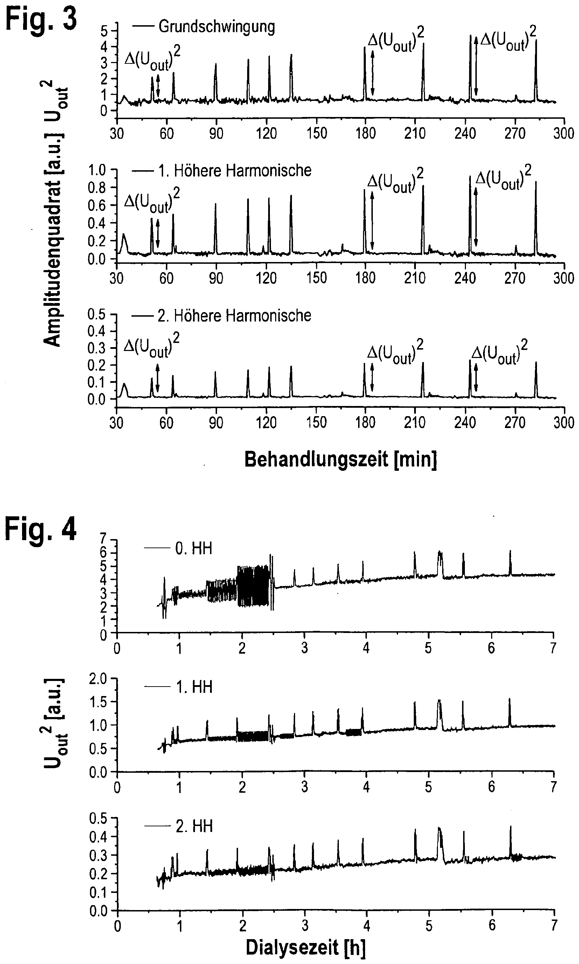

Fig. 3 zeigt das Amplitudenquadrat $U_{out}^2$ der Grundschwingung sowie der ersten und zweiten höheren Harmonischen des Drucksignals des Drucksensors 33 als Funktion der Behandlungszeit, wobei während der gesamten Behandlung die Substituatpumpe 24 in vorgegebenen Zeitabschnitten kurzzeitig angehalten wird. Zu dem Zeitpunkt, wo die Substituatpumpe angehalten wird, erhöhen sich die Amplituden der Grundschwingung sowie der Oberschwingungen des Drucksignals. Dies ist deutlich in Fig. 3 zu erkennen. Die Auswert- und Recheneinheit 32 bestimmt durch Differenzbildung vor und nach der Änderung der Substituat- und Ultrafiltrationsrate die Höhe der Amplitudenänderung und berechnet das Quadrat der Amplitudenänderung $\Delta U_{out}^2$.

Nach Gleichung (5) berechnet die Rechen- und Auswerteinheit 32 aus dem Amplitudenquadrat $U^2_{out}$ und der Änderung des Amplitudenquadrats $\Delta U^2_{out}$ die Änderung des Strömungswiderstandes $(R+\Delta R)/R$.

Die Rechen- und Auswerteinheit 32 verfügt über eine Vergleichseinheit 32B, die den berechneten Wert für die Strömungswiderstandsänderung $(R+\Delta R)/R$ mit einem vorgegebenen Grenzwert vergleicht. Wenn die Änderung des Strömungswiderstandes den Grenzwert überschreitet, steuert die Rechen- und Auswerteinheit 32 die Steuer- und Recheneinheit 25 der Dialysevorrichtung an, die einen akustischen und/oder optischen Alarm auslösen und/oder einen Eingriff in die Maschinensteuerung vornehmen kann, um ein Zusetzen der Membran 2 des Dialysators 1 zu verhindern. Mögliche Gegenmaßnahmen sind beispielsweise eine Verringerung der Ultrafiltrationsrate, wodurch der Verdickung des Bluts entgegengewirkt wird.

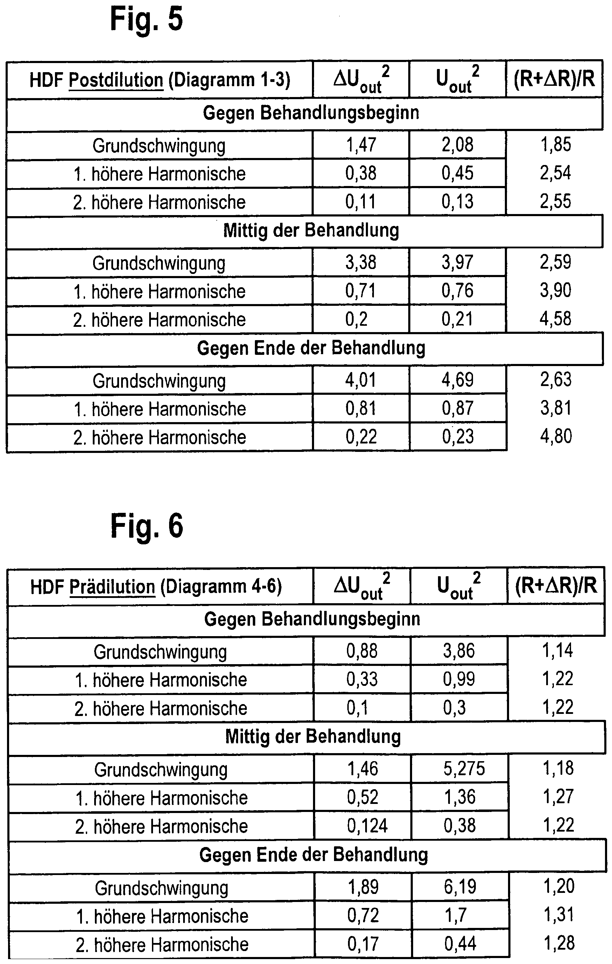

Aus der Tabelle von Fig. 5 sind die berechneten Größen $U_{out}^2$ und $\Delta U_{out}^2$ sowie die Änderung des Strömungswiderstandes $(R+\Delta R)/R$ zu Beginn der Blutbehandlung, in der Mitte der Blutbehandlung und zum Ende der Blutbehandlung für die Grundschwingung sowie die erste und zweite Harmonische für den Fall der Postdilution ersichtlich.

Die spektral zerlegten Beiträge des venösen Drucksignals während der Blutbehandlung sind ein direktes Maß für den Strömungswiderstand des Dialysators längs der Dialysatorfasern. Bei steigendem Strömungswiderstand steigt die Gefahr des Faserverschlusses sowie der Hämolyse während der Behandlung. Insbesondere bei der Hämodiafiltration mit Postdilution steigt der Strömungswiderstand längs der Dialysatorfasern während der Behandlung oft unbemerkt stark an, so dass der Dialysator zu klotten beginnen und der Dialysatoreingangsdruck kritische Werte erreichen kann. Das erfindungsgemäße Verfahren erlaubt die Zunahme des Strömungswiderstandes während der Hämodiafiltrationsbehandlung abzuschätzen, so dass Gegenmaßnahmen getroffen werden können, um den Strömungswiderstand konstant zu halten oder zu verringern.

Nachfolgend wird angenommen, dass die Hämodiafiltrationsvorrichtung mit Prädilution betrieben wird, wobei die Substituatpumpe 24 stillsteht und die Substituatpumpe 22 läuft, so dass Substituat stromauf des Dialysators 1 dem Blutkreislauf 9 zugeführt wird. Die Rechen- und Auswerteinheit analysiert weiterhin das Drucksignal des Drucksensors 33.

Fig. 4 zeigt die Amplitudenquadrate $U_{out}^2$ der Grundschwingung sowie der ersten und zweiten höheren Harmonischen als Funktion der Behandlungszeit bei der Hämodiafiltration mit Prädilution. Es zeigt sich, dass die Amplitude des Drucksignals der Grundschwingung sowie der ersten und zweiten Oberschwingung zunimmt, wenn die Substituatpumpe 22 kurzzeitig angehalten wird. Allerdings sind die Effekte nicht so deutlich wie bei der Postdilution, da das in die Blutkammer 3 des Dialysators 1 strömende Blut bereits eine geringere Viskosität hat, so dass eine anschließende Verdickung keinen so großen Einfluss mehr auf die Viskosität des strömenden Bluts ausüben kann.

Die Tabelle von Fig. 6 zeigt die berechneten Größen $\Delta U_{out}^2$ und $U_{out}^2$ sowie die Änderung des Strömungswiderstandes $(R+\Delta R)/R$ zu Beginn der Hämodiafiltration mit Prädilution, in der Mitte der Behandlung und zum Ende der Behandlung für die Grundschwingung sowie die erste und zweite höhere Harmonische.

## Patentansprüche

1.  Vorrichtung zur Überwachung der Zunahme des Strömungswiderstandes einer durch eine semipermeable Membran in eine Blutkammer (3) und eine Dialysierflüssigkeitskammer (4) unterteilten Blutbehandlungseinheit (1) für eine extrakorporale Blutbehandlungsvorrichtung während einer extrakorporalen Blutbehandlung,
    wobei die extrakorporale Blutbehandlungsvorrichtung aufweist:

    einen extrakorporalen Blutkreislauf (9) mit einem arteriellen Zweig (20), der zu der Blutkammer der Blutbehandlungseinheit führt, und einen von der Blutkammer abgehenden venösen Zweig (21), und ein Dialysierflüssigkeitssystem (10), in dem die Dialysierflüssigkeitskammer angeordnet ist,
    eine Ultrafiltrationseinrichtung (18) zum Entziehen von Ultrafiltrat mit einer vorgegebenen Ultrafiltrationsrate dem im extrakorporalen Blutkreislauf strömenden Blut über die semipermeable Membran (2) der Blutbehandlungseinheit (1), und
    eine Einrichtung (19) zum Zuführen von Substituat mit einer vorgegebenen Substituatrate stromauf oder stromab der Blutbehandlungseinheit dem im extrakorporalen Blutkreislauf strömenden Blut,
    wobei die Vorrichtung zur Überwachung der Blutbehandlungseinheit aufweist:

    Mittel (33, 34; 32) zum Messen von oszillierenden Drucksignalen im extrakorporalen Blutkreislauf oder im Dialysierflüssigkeitssystem, und Mittel (32) zum Analysieren der im extrakorporalen Blutkreislauf oder im Dialysierflüssigkeitssystem gemessenen Drucksignale,
    wobei
    die Mittel (33, 34; 32) zum Messen der oszillierenden Drucksignale derart ausgebildet sind,
    dass im extrakorporalen Blutkreislauf oder im Dialysierflüssigkeitssystem ein oszillierendes Drucksignal zu einem Zeitpunkt gemessen wird, wenn dem extrakorporalen Blutkreislauf Substituat mit einer vorgegebenen ersten Substituatrate bzw. kein Substituat stromauf oder stromab der Blutbehandlungseinheit zugeführt wird, und/oder über die semipermeable Membran der Blutbehandlungseinheit Ultrafiltrat mit einer vorgegebenen ersten Ultrafiltrationsrate bzw. kein Ultrafiltrat entzogen wird, und
    dass im extrakorporalen Blutkreislauf oder im Dialysierflüssigkeitssystem ein oszillierendes Drucksignal zu einem Zeitpunkt gemessen wird, wenn dem extrakorporalen Blutkreislauf Substituat mit einer vorgegebenen zweiten Substituatrate bzw. kein Substituat zugeführt wird, wobei sich die zweite Substituatrate von der ersten Substituatrate unterscheidet, und/oder über die Membran der Blutbehandlungseinheit Ultrafiltrat mit einer vorgegebenen zweiten Ultrafiltrationsrate bzw. kein Ultrafiltrat entzogen wird, wobei sich die zweite Ultrafiltrationsrate von der ersten Ultrafiltrationsrate unterscheidet,
    wobei zur Überwachung der Zunahme des Strömungswiderstandes während der Blutbehandlung die Mittel (32) zum Analysieren derart ausgebildet sind, dass auf der Grundlage der in einem ersten vorausgehenden Zeitintervall der Blutbehandlung gemessenen oszillierenden Drucksignale vor und nach der Änderung der Substituatrate und/oder Ultrafiltrationsrate eine mit der Änderung des Strömungswiderstandes der Blutbehandlungseinheit (1) korrelierende erste Größe berechnet wird, und

    **dadurch gekennzeichnet, dass** auf der Grundlage der in einem zweiten nachfolgenden Zeitintervall der Blutbehandlung gemessenen oszillierenden Drucksignale vor und nach der Änderung der Substituatrate und/oder Ultrafiltrationsrate eine mit der Änderung des Strömungswiderstandes der Blutbehandlungseinheit (1) korrelierende zweite Größe berechnet wird, wobei
    die erste und zweite Größe zur Abschätzung der Zunahme des Strömungswiderstandes der Blutbehandlungseinheit (1) während der Blutbehandlung ausgewertet werden.

2.  Vorrichtung nach Anspruch 1, **dadurch gekennzeichnet, dass** die Mittel (33, 34; 32) zum Messen der oszillierenden Drucksignale weiterhin derart ausgebildet sind, dass zu dem Zeitpunkt, zu dem Substituat mit einer vorgegebenen ersten Substituatrate zugeführt wird, dem extrakorporalen Blutkreislauf (9) eine vorgegebene Menge an Ultrafiltrat mit einer vorgegebenen ersten Ultrafiltrationsrate entzogen wird, und zu dem Zeitpunkt, zu dem Substituat mit einer vorgegebenen zweiten Substituatrate zugeführt oder kein Substituat zugeführt wird, dem extrakorporalen Blutkreislauf eine vorgegebenen Menge an Ultrafiltrat mit einer vorgegebenen zweiten Ultrafiltrationsrate entzogen wird, die sich von der ersten Ultrafiltrationsrate unterscheidet.

3. Vorrichtung nach Anspruch 2, **dadurch gekennzeichnet, dass** die Ultrafiltrationsrate um den gleichen Betrag erhöht bzw. verringert ist, wie die Substituatrate erhöht bzw. verringert.

4. Vorrichtung nach einem der Ansprüche 1 bis 3, **dadurch gekennzeichnet, dass** die Mittel (32) zum Analysieren des oszillierenden Drucksignals derart ausgebildet sind, dass die mit der Änderung des Strömungswiderstandes korrelierende Größe mit einem vorgegebenen Grenzwert verglichen wird, wobei auf einen kritischen Zustand geschlossen wird, wenn die mit der Änderung des Strömungswiderstandes korrelierende Größe den vorgegebenen Grenzwert überschreitet.

5. Vorrichtung nach einem der Ansprüche 1 bis 4, **dadurch gekennzeichnet, dass** die Mittel (32) zum Analysieren des oszillierenden Drucksignals derart ausgebildet sind, dass das Frequenzspektrum des vor der Änderung der Substituatrate gemessenen oszillierenden Drucksignals und des nach der Änderung der Substituatrate gemessenen oszillierenden Drucksignals analysiert wird, und die Änderung der Amplitude der Grundschwingung und/oder die Änderung der Amplitude mindestens einer Oberschwingung des vor und nach der Änderung Substituatrate gemessenen oszillierenden Drucksignals bestimmt wird, und auf der Grundlage der Änderung der Amplitude die Änderung des Strömungswiderstandes berechnet wird.

6. Vorrichtung nach Anspruch 5, **dadurch gekennzeichnet, dass** die Mittel (32) zum Analysieren des oszillierenden Drucksignals derart ausgebildet sind, dass das Quadrat der Amplitude $U^2(\omega)$ der Grundschwingung und/oder einer Oberschwingung des gemessenen oszillierenden Drucksignals vor der Änderung der Substituatrate und das Quadrat der Änderung $\Delta U^2(\omega)$ der Amplitude der Grundschwingung und/oder einer Oberschwingung nach der Änderung der Substituatrate berechnet werden, und die Änderung des Strömungswiderstandes R nach der folgenden Gleichung berechnet wird:

$$\frac{R + \Delta R}{R} \approx \sqrt{\frac{U^2_{out}(\omega)}{U^2_{out}(\omega) - \Delta U^2_{out}(\omega)}}$$

7. Vorrichtung nach Anspruch 5 oder 6, **dadurch gekennzeichnet, dass** die Mittel (32) zum Analysieren des oszillierenden Drucksignals Mittel (32A) zur Durchführung einer Fourier-Transformation aufweisen.

8. Blutbehandlungsvorrichtung mit einer Vorrichtung nach einem der Ansprüche 1 bis 7, wobei die Blutbehandlungsvorrichtung aufweist:

einen extrakorporalen Blutkreislauf (9) mit einem arteriellen Zweig (20), der zu der Blutkammer (3) der Blutbehandlungseinheit (1) führt, und einen von der Blutkammer abgehenden venösen Zweig (21), und ein Dialysierflüssigkeitssystem (10), in dem die Dialysierflüssigkeitskammer (4) angeordnet ist,
eine Ultrafiltrationsvorrichtung (18) zum Entziehen von Ultrafiltrat mit einer vorgegebenen Ultrafiltrationsrate dem im extrakorporalen Blutkreislauf strömenden Blut über die semipermeable Membran (2) der Blutbehandlungseinheit (1), und
eine Einrichtung (37) zum Zuführen von Substituat mit einer vorgegebenen Substituatrate stromauf oder stromab der Blutbehandlungseinheit dem im extrakorporalen Blutkreislauf strömenden Blut.

9. Blutbehandlungsvorrichtung nach Anspruch 8, **dadurch gekennzeichnet, dass** die Blutbehandlungsvorrichtung eine im arteriellen Zweig (20) des extrakorporalen Blutkreislaufs (9) angeordneten Blutpumpe (6), die ein oszillierendes Drucksignal erzeugt, insbesondere eine okkludierende Blutpumpe, aufweist.

10. Blutbehandlungsvorrichtung nach Anspruch 8 oder 9, **dadurch gekennzeichnet, dass** die Mittel (33, 34; 32) zum Messen des oszillierenden Drucksignals einen den Druck im venösen Zweig (21) des extrakorporalen Blutkreislaufs messenden Drucksensor (33) aufweisen.

**11.** Blutbehandlungsvorrichtung nach einem der Ansprüche 8 bis 10, **dadurch gekennzeichnet, dass** das Dialysierflüssigkeitssystem (9) eine zu der Dialysierflüssigkeitskammer (4) der Blutbehandlungseinheit (1) führende Dialysierflüssigkeitszuführleitung (12) und eine von der Dialysierflüssigkeitskammer abgehende Dialysierflüssigkeitsabführleitung (13) aufweist, wobei die Mittel (33, 34; 32) zum Messen des oszillierenden Drucksignals einen den Druck in der Dialysierflüssigkeitsabführleitung (13) messenden Drucksensor (34) aufweisen.

## Claims

**1.** A device for monitoring the increase in the flow resistance of a blood treatment unit (1) for an extracorporeal blood treatment apparatus during an extracorporeal blood treatment, said blood treatment unit being divided by a semipermeable membrane into a blood chamber (3) and a dialysing fluid chamber (4), whereby the extracorporeal blood treatment apparatus comprises:

an extracorporeal blood circuit (9) with an arterial branch (20), which leads to the blood chamber of the blood treatment unit, and a venous branch (21) leading away from the blood chamber, and a dialysing fluid system (10) in which the dialysing fluid chamber is disposed, an ultrafiltration device (18) for withdrawing ultrafiltrate at a preset ultrafiltration rate from the blood flowing in the extracorporeal blood circuit via the semipermeable membrane (2) of the blood treatment unit (1), and a device (19) for supplying substituate at a preset substituate rate upstream or downstream of the blood treatment unit to the blood flowing in the extracorporeal blood circuit, whereby the device for monitoring the blood treatment unit comprises:

means (33, 34; 32) for measuring the oscillating pressure signals in the extracorporeal blood circuit or in the dialysing fluid system, and means (32) for analysing the pressure signals measured in the extracorporeal blood circuit or in the dialysing fluid system,

whereby the means (33, 34; 32) for measuring the oscillating pressure signals are configured in such a way that, in the extracorporeal blood circuit or in the dialysing fluid system, an oscillating pressure signal is measured at a time when substituate is fed at a preset first substituate rate or no substituate is fed upstream or downstream of the blood treatment unit to the extracorporeal blood circuit, and/or ultrafiltrate is withdrawn at a preset first ultrafiltration rate or no ultrafiltrate is withdrawn via the semipermeable membrane of the blood treatment unit, and in the extracorporeal blood circuit or in the dialysing fluid system, an oscillating pressure signal is measured at a time when substituate is fed at a preset second substituate rate or no substituate is fed to the extracorporeal blood circuit, the second substituate rate differing from the first substituate rate, and/or ultrafiltrate is withdrawn at a preset second ultrafiltration rate or no ultrafiltrate is withdrawn via the membrane of the blood treatment unit, the second ultrafiltration rate differing from the first ultrafiltration rate, whereby, for monitoring the increase in the flow resistance during blood treatment the means (32) for analysing are configured in such a way that, on the basis of the measured oscillating pressure signals in a first preceding time interval of the blood treatment before and after the change in the substituate rate and/or the ultrafiltration rate, a first quantity correlating with the change in the flow resistance of the blood treatment unit (1) is calculated, and **characterized in that**, on the basis of the measured oscillating pressure signals in a second following time interval of the blood treatment before and after the change in the substituate rate and/or the ultrafiltration rate, a second quantity correlating with the change in the flow resistance of the blood treatment unit (1) is calculated, whereby the first and the second quantity are evaluated for assessing the increase in flow resistance of the blood treatment unit (1) during the blood treatment.

**2.** The device according to claim 1, **characterised in that** the means (33, 34; 32) for measuring the oscillating pressure signals are further configured in such a way that, at the time when substituate is fed at a preset first substituate rate, a preset quantity of ultrafiltrate is withdrawn at a preset first ultrafiltration rate from the extracorporeal blood circuit (9), and at the time when substituate is fed at a preset second substituate rate or no substituate is fed, a preset quantity of ultrafiltrate is withdrawn from the extracorporeal blood circuit at a preset second ultrafiltration rate which differs from the first ultrafiltration rate.

3. The device according to claim 2, **characterised in that** the ultrafiltration rate is increased or reduced by the same amount as the substitute rate is increased or reduced.

4. The device according to any one of claims 1 to 3, **characterised in that** the means (32) for analysing the oscillating pressure signal are configured in such a way that the quantity correlating with the change in the flow resistance is compared with a preset threshold value, it being concluded that there is a critical state if the quantity correlating with the change in the flow resistance exceeds the preset threshold value.

5. The device according to any one of claims 1 to 4, **characterised in that** the means (32) for analysing the oscillating pressure signal are configured in such a way that the frequency spectrum of the oscillating pressure signal measured before the change in the substitute rate and of the oscillating pressure signal measured after the change in the substitute rate is analysed, and the change in the amplitude of the fundamental oscillation and/or the change in the amplitude of at least one harmonic of the oscillating pressure signal measured before and after the change in the substitute rate is determined, and the change in the flow resistance is calculated on the basis of the change in the amplitude.

6. The device according to claim 5, **characterised in that** the means (32) for analysing the oscillating pressure signal are configured in such a way that the square of the amplitude $U^2(\omega)$ of the fundamental oscillation and/or a harmonic of the measured oscillating pressure signal is calculated before the change in the substitute rate and the square of the change $\Delta U^2(\omega)$ in the amplitude of the fundamental oscillation and/or a harmonic is calculated after the change in the substitute rate, and the change in the flow resistance R is calculated according to the following equation:

$$\frac{R + \Delta R}{R} \approx \sqrt{\frac{U_{out}^2(\omega)}{U_{out}^2(\omega) - \Delta U_{out}^2(\omega)}}$$

7. The device according to claim 5 or 6, **characterised in that** the means (32) for analysing the oscillating pressure signal comprise means (32A) for performing a Fourier transform.

8. A blood treatment apparatus with a device according to any one of claims 1 to 7, wherein the blood treatment apparatus comprises:

   an extracorporeal blood circuit (9) with an arterial branch (20), which leads to the blood chamber (3) of the blood treatment unit (1), and a venous branch (21) leading away from the blood chamber, and a dialysing fluid system (10) in which the dialysing fluid chamber (4) is disposed,
   an ultrafiltration device (18) for withdrawing ultrafiltrate at a preset ultrafiltration rate from the blood flowing in the extracorporeal blood circuit via the semipermeable membrane (2) of the blood treatment unit (1), and
   a device (37) for supplying substitute at a preset substitute rate upstream or downstream of the blood treatment unit to the blood flowing in the extracorporeal blood circuit.

9. The blood treatment apparatus according to claim 8, **characterised in that** the blood treatment apparatus comprises a blood pump (6), which is arranged in the arterial branch (20) of the extracorporeal blood circuit (9) and which generates an oscillating pressure signal, in particular an occluding blood pump.

10. The blood treatment apparatus according to claim 8 or 9, **characterised in that** the means (33, 34; 32) for measuring the oscillating pressure signal comprise a pressure sensor (33) which measures the pressure in the venous branch (21) of the extracorporeal blood circuit.

11. The blood treatment apparatus according to any one of claims 8 to 10, **characterised in that** the dialysing fluid system (9) comprises a dialysing fluid supply line (12) leading to the dialysing fluid chamber (4) of the blood treatment unit (1) and a dialysing fluid discharge line (13) leading away from the dialysing fluid chamber, the means (33, 34; 32) for measuring the oscillating pressure signal comprising a pressure sensor (34) which measures the pressure in the dialysing fluid discharge line (13).

**Revendications**

1. Dispositif de surveillance de l'augmentation de la résistance à l'écoulement d'une unité de traitement du sang divisée par une membrane semi-perméable en une chambre pour le sang (3) et en une chambre pour le liquide de dialyse (4) pour un dispositif de traitement du sang extracorporel pendant un traitement du sang extracorporel, le dispositif de traitement du sang extracorporel présentant :

   un circuit de sang extracorporel (9) avec une dérivation artérielle (20) qui conduit à la chambre pour le sang de l'unité de traitement du sang, et une dérivation veineuse (21) partant de la chambre pour le sang, et un système de liquide de dialyse (10) dans lequel est disposée la chambre pour le liquide de dialyse, un équipement d'ultrafiltration (18) destiné à prélever de l'ultrafiltrat, avec un taux d'ultrafiltration spécifié au préalable, dans le sang s'écoulant dans le circuit de sang extracorporel par le biais de la membrane semi-perméable (2) de l'unité de traitement du sang (1), et un équipement (19) destiné à l'acheminement de liquide de substitution, avec un taux de liquide de substitution spécifié au préalable, en amont ou en aval de l'unité de traitement du sang, vers le sang s'écoulant dans le circuit de sang extracorporel,

   le dispositif de surveillance de l'unité de traitement du sang présentant :

   des moyens (33, 34 ; 32) destinés à la mesure de signaux de pression oscillants dans le circuit de sang extracorporel ou dans le système de liquide de dialyse, et des moyens (32) destinés à l'analyse des signaux de pression mesurés dans le circuit de sang extracorporel ou dans le système de liquide de dialyse,

   les moyens (33, 34 ; 32) destinés à la mesure des signaux de pression oscillants étant constitués de telle sorte que, dans le circuit de sang extracorporel ou dans le système de liquide de dialyse, un signal de pression oscillant est mesuré à un moment où du liquide de substitution est acheminé au circuit de sang extracorporel avec un premier taux de liquide de substitution spécifié au préalable ou respectivement quand aucun liquide de substitution n'est acheminé en amont ou en aval de l'unité de traitement du sang, et/ou de l'ultrafiltat est prélevé, par le biais de la membrane semi-perméable de l'unité de traitement du sang, avec un premier taux d'ultrafiltration spécifié au préalable ou respectivement quand aucun ultrafiltrat n'est prélevé, et un signal de pression oscillant est mesuré dans le circuit de sang extracorporel ou dans le système de liquide de dialyse à un moment où du liquide de substitution est acheminé au circuit de sang extracorporel avec un deuxième taux de liquide de substitution spécifié au préalable ou respectivement quand aucun liquide de substitution n'est acheminé, le deuxième taux de liquide de substitution étant différent du premier taux de liquide de substitution, et/ou de l'ultrafiltrat étant prélevé par le biais de la membrane de l'unité de traitement du sang avec un deuxième taux d'ultrafiltration spécifié au préalable ou respectivement aucun ultrafiltrat n'étant prélevé, le deuxième taux d'ultrafiltration étant différent du premier taux d'ultrafiltration, les moyens (32) destinés à l'analyse étant, pour la surveillance de l'augmentation de la résistance à l'écoulement pendant le traitement du sang, constitués de telle sorte que, sur la base des signaux de pression oscillants mesurés dans un premier intervalle de temps précédent de traitement du sang avant et après la variation du taux de liquide de substitution et/ou du taux d'ultrafiltration, une première grandeur en corrélation avec la variation de la résistance à l'écoulement de l'unité de traitement du sang (1) est calculée, et **caractérisé en ce que**, sur la base des signaux de pression oscillants mesurés dans un deuxième intervalle de temps suivant du traitement du sang avant et après la variation du taux de liquide de substitution et/ou du taux d'ultrafiltration, une deuxième grandeur en corrélation avec la variation de la résistance à l'écoulement de l'unité de traitement du sang (1) est calculée, la première et la deuxième grandeur étant analysées pour l'estimation de l'augmentation de la résistance à l'écoulement de l'unité de traitement du sang (1) pendant le traitement du sang.

2. Dispositif selon la revendication 1, **caractérisé en ce que** les moyens (33, 34 ; 32) destinés à la mesure des signaux de pression oscillants sont en outre constitués de telle sorte que, au moment où du liquide de substitution est acheminé avec un premier taux de liquide de substitution spécifié au préalable, une quantité spécifiée au préalable d'ultrafiltrat est prélevé dans le circuit de sang extracorporel (9) avec un premier taux d'ultrafiltration spécifié au préalable et, au moment où du liquide de substitution est acheminé avec une deuxième taux de liquide de substitution spécifié au préalable ou aucun liquide de substitution n'est acheminé, une quantité d'ultrafiltrat spécifiée au préalable est prélevée dans le circuit de sang extracorporel avec un deuxième taux d'ultrafiltration spécifié au préalable qui est différent du premier taux d'ultrafiltration.

3. Dispositif selon la revendication 2, **caractérisé en ce que** le taux d'ultrafiltration est augmenté ou respectivement réduit d'une valeur identique à celle de l'augmentation ou respectivement de la réduction du taux de liquide de substitution.

4. Dispositif selon l'une des revendications 1 à 3, **caractérisé en ce que** les moyens (32) destinés à l'analyse du signal de pression oscillant sont constitués de telle sorte que la grandeur en corrélation avec la variation de la résistance à l'écoulement est comparée à une valeur limite spécifiée au préalable, un état critique étant présumé si la grandeur en corrélation avec la variation de la résistance à l'écoulement est supérieure à la valeur limite spécifiée au préalable.

5. Dispositif selon l'une des revendications 1 à 4, **caractérisé en ce que** les moyens (32) destinés à l'analyse du signal de pression oscillant sont constitués de telle sorte que le spectre de fréquences du signal de pression oscillant mesuré avant la variation du taux de liquide de substitution et du signal de pression oscillant mesuré après la variation du taux de liquide de substitution est analysé, et la variation de l'amplitude de l'oscillation fondamentale et/ou la variation de l'amplitude d'au moins une oscillation harmonique du signal de pression oscillant mesuré avant et après la variation du taux de liquide de substitution est définie, et la variation de la résistance à l'écoulement est calculée sur la base de la variation de l'amplitude.

6. Dispositif selon la revendication 5, **caractérisé en ce que** les moyens (32) destinés à l'analyse du signal de pression oscillant sont constitués de telle sorte que le carré de l'amplitude $U^2(\omega)$) de l'oscillation fondamentale et/ou d'une oscillation harmonique du signal de pression oscillant mesuré avant la variation du taux de liquide de substitution et le carré de la variation $\Delta U^2(\omega)$ de l'amplitude de l'oscillation fondamentale et/ou d'une oscillation harmonique après la variation du taux de liquide de substitution sont calculés, et la variation de la résistance à l'écoulement R est calculée selon l'équation suivante :

$$\frac{R + \Delta R}{R} \approx \sqrt{\frac{U_{out}^2(\omega)}{U_{out}^2(\omega) - \Delta U_{out}^2(\omega)}}$$

7. Dispositif selon la revendication 5 ou 6, **caractérisé en ce que** les moyens (32) destinés à l'analyse du signal de pression oscillant présentent des moyens (32A) destinés à la réalisation d'une transformation de Fourier.

8. Dispositif de traitement du sang avec un dispositif selon l'une des revendications 1 à 7, le dispositif de traitement du sang présentant :

un circuit de sang extracorporel (9) avec une dérivation artérielle (20) qui conduit à la chambre pour le sang (3) de l'unité de traitement du sang (1), et une dérivation veineuse (21) partant de la chambre pour le sang et un système de liquide de dialyse (10) dans lequel est disposée la chambre pour le liquide de dialyse (4),
un équipement d'ultrafiltration (18) destiné au prélèvement d'ultrafiltrat, avec un taux d'ultrafiltration spécifié au préalable, dans le sang circulant dans le circuit de sang extracorporel par le biais de la membrane semi-perméable (2) de l'unité de traitement du sang (1), et
un équipement (37) destiné à l'acheminement de liquide de substitution, avec un taux de liquide de substitution spécifié au préalable, en amont ou en aval de l'unité de traitement du sang, vers le sang circulant dans le circuit de sang extracorporel.

9. Dispositif de traitement du sang selon la revendication 8, **caractérisé en ce que** le dispositif de traitement du sang présente une pompe à sang (6), en particulier une pompe à sang occlusive, qui est disposée dans la dérivation artérielle (20) du circuit de sang extracorporel (9) et qui produit un signal de pression oscillant (10).

10. Dispositif de traitement du sang selon la revendication 8 ou 9, **caractérisé en ce que** les moyens (33, 34 ; 32) destinés à la mesure du signal de pression oscillant présentent un capteur de pression (33) mesurant la pression dans la dérivation veineuse (21) du circuit de sang extracorporel.

11. Dispositif de traitement du sang selon l'une des revendications 8 à 10, **caractérisé en ce que** le système de liquide de dialyse (9) présente une conduite d'acheminement de liquide de dialyse (12) conduisant à la chambre pour le liquide de dialyse (4) de l'unité de traitement du sang (1) et une conduite d'évacuation de liquide de dialyse (13) partant de la chambre pour le liquide de dialyse, les moyens (33, 34 ; 32) destinés à la mesure du signal de pression oscillant présentant un capteur de pression (34) mesurant la pression dans la conduite d'évacuation de liquide de dialyse (13).

# Fig. 1

# Fig. 2

Fig. 3

Fig. 4

# Fig. 5

| HDF Postdilution (Diagramm 1-3) | $\Delta U_{out}^2$ | $U_{out}^2$ | $(R+\Delta R)/R$ |
|---|---|---|---|
| Gegen Behandlungsbeginn | | | |
| Grundschwingung | 1,47 | 2,08 | 1,85 |
| 1. höhere Harmonische | 0,38 | 0,45 | 2,54 |
| 2. höhere Harmonische | 0,11 | 0,13 | 2,55 |
| Mittig der Behandlung | | | |
| Grundschwingung | 3,38 | 3,97 | 2,59 |
| 1. höhere Harmonische | 0,71 | 0,76 | 3,90 |
| 2. höhere Harmonische | 0,2 | 0,21 | 4,58 |
| Gegen Ende der Behandlung | | | |
| Grundschwingung | 4,01 | 4,69 | 2,63 |
| 1. höhere Harmonische | 0,81 | 0,87 | 3,81 |
| 2. höhere Harmonische | 0,22 | 0,23 | 4,80 |

# Fig. 6

| HDF Prädilution (Diagramm 4-6) | $\Delta U_{out}^2$ | $U_{out}^2$ | $(R+\Delta R)/R$ |
|---|---|---|---|
| Gegen Behandlungsbeginn | | | |
| Grundschwingung | 0,88 | 3,86 | 1,14 |
| 1. höhere Harmonische | 0,33 | 0,99 | 1,22 |
| 2. höhere Harmonische | 0,1 | 0,3 | 1,22 |
| Mittig der Behandlung | | | |
| Grundschwingung | 1,46 | 5,275 | 1,18 |
| 1. höhere Harmonische | 0,52 | 1,36 | 1,27 |
| 2. höhere Harmonische | 0,124 | 0,38 | 1,22 |
| Gegen Ende der Behandlung | | | |
| Grundschwingung | 1,89 | 6,19 | 1,20 |
| 1. höhere Harmonische | 0,72 | 1,7 | 1,31 |
| 2. höhere Harmonische | 0,17 | 0,44 | 1,28 |

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10355042 B3
- WO 2004073772 A1
- US 20020174721 A1
- US 6623443 B1
- WO 03047656 A1
- EP 1595560 A1
- EP 0330761 A1
- DE 19734002 C1